# FASCICULE DE BREVET EUROPEEN

(11) **EP 4 340 935 B1**
(45) Date de publication et mention de la délivrance du brevet: **02.10.2024**
(21) Numéro de dépôt: 22762127.3
(22) Date de dépôt: 29.07.2022
(51) Int. Cl.: A61N 1/36, A61N 1/05

(54) **STIMULATION NEURALE ENTRELACÉE**
VERSCHACHTELTE NERVENSTIMULATION
INTERLEAVED NEURAL STIMULATION

(30) Priorité: 30.07.2021 FR 2108317
(43) Date de publication de la demande: 27.03.2024
(73) Titulaire: Neurinnov, 34000 Montpellier (FR); INRIA - Institut National de Recherche en Informatique et en Automatique, 78150 Le Chesnay (FR); Université de Montpellier, 34090 Montpellier (FR)
(72) Inventeur: GUIRAUD, David, 34095 Montpellier (FR); ANDREU, David, 34095 Montpellier (FR)
(74) Mandataire: Icosa
(86) Numéro de dépôt international: PCT/FR2022/051524
(87) Numéro de publication internationale: WO 2023/007100

(56) Documents cités:
- US-A- 5 861 017
- US-A1- 2018 178 008
- TIGRA WAFA ET AL: "Selective neural electrical stimulation restores hand and forearm movements in individuals with complete tetraplegia", JOURNAL OF NEUROENGINEERING AND REHABILITATION, vol. 17, no. 1, 1 December 2020 (2020-12-01), XP055927891, DOI: 10.1186/s12984-020-00676-4
- KATHARINE H POLASEK ET AL: "Human Nerve Stimulation Thresholds and Selectivity Using a Multi-contact Nerve Cuff Electrode", IEEE TRANSACTIONS ON NEURAL SYSTEMS AND REHABILITATION ENGINEERING, IEEE, USA, vol. 15, no. 1, 1 March 2007 (2007-03-01), pages 76 - 82, XP011174735, ISSN: 1534-4320, [retrieved on 20220610], DOI: 10.1109/TNSRE.2007.891383

## Description

### DOMAINE DE L'INVENTION

La présente invention concerne les stimulateurs neuraux, en particulier les stimulateurs destinés à la restauration du mouvement de membres paralysés. Ces stimulateurs sont destinés à activer un ou plusieurs muscles par excitation d'un nerf au moyen d'un stimulus électrique.

### ÉTAT DE LA TECHNIQUE

La proportion de personnes atteintes de tétraplégie suite à un traumatisme médullaire ou cervical est en constante croissance. Améliorer la qualité de vie de ces personnes est un défi, et la restauration de la capacité de préhension est une priorité, tant l'utilisation des mains est un facteur clé de la qualité de la vie quotidienne. Différentes approches ont été développées pour restaurer les fonctions de la main, du poignet et de l'avant-bras, qui concourent au mécanisme de préhension. Des méthodes chirurgicales - transferts musculo-tendineux ou transplantations nerveuses - sont envisageables dans les cas où un nombre suffisant de muscles est encore contrôlé de manière volontaire par la personne tétraplégique. Alternativement, il est possible d'utiliser des méthodes de stimulations électriques, soit appliquées directement aux muscles - ce qui requiert des dispositifs invasifs et nombreux - soit appliquées sur des nerfs intacts en aval de la lésion. Cette dernière méthode consiste à placer des électrodes sur les nerfs assurant l'innervation des muscles contrôlant la main et le poignet, en particulier : le nerf médian, le nerf ulnaire (ou cubital) et la branche superficielle du nerf radial. Les deux approches, stimulation musculaire et neurale, peuvent être combinées.

Il est désormais connu de stimuler sélectivement les nerfs médian et radial afin de restaurer des mouvements élémentaires comme la prise par opposition subterminale (pince), la prise penta-digitale palmaire (sphérique) ou la prise à pleine main par exemple - voir Sélective neural electrical stimulation restores hand and forearm movements in individuals with complete tetraplegia ; Tigra et al. *Journal of NeuroEngineering and Réhabilitation* (2020) 17:66 ; DOI : 10.1186/s12984-020-00676-4.

Toutefois, ces méthodes de stimulation demeurent incomplètes car elles ne permettent pas de restaurer les mouvements de préhension dans toute leur complexité. La présente invention vise à dépasser ces limites en proposant un dispositif de stimulation appliquant des stimulations, notamment neurales, plus complexes.

### RÉSUMÉ

L'invention concerne donc un dispositif de stimulation neurale comprenant
- au moins une électrode neurale comportant au moins deux contacts électriques et destinée à être implantée sur au moins un nerf ;
- un générateur d'impulsions de courant connecté à la au moins une électrode neurale par l'intermédiaire d'un répartiteur de courant ;
- un moyen de stockage configuré pour contenir une librairie de séquences, chaque séquence comprenant une série temporelle d'impulsions de courant associée à une configuration de contacts électriques ; et
- un séquenceur configuré pour appliquer une activation neurale à la au moins une électrode neurale, ladite activation neurale comprenant au moins deux séquences entrelacées.

Dans un mode de réalisation, les séquences à entrelacer comprennent le même nombre d'intervalles, ces intervalles ayant la même durée.

Dans un mode de réalisation, le dispositif de stimulation neurale comprend en outre un contrôleur configuré pour piloter une succession d'activations neurales. En particulier, le contrôleur configure le séquenceur puis active les séquences à entrelacer.

Dans un mode de réalisation, la au moins une électrode neurale comprend une pluralité de configurations de contacts électriques.

Dans un mode de réalisation, les impulsions de courant sont des stimuli biphasiques équilibrés en charge. De préférence, la première phase des stimuli biphasiques équilibrés en charge a une amplitude comprise entre 10 µA et 6000 µA et une durée comprise entre 5 µs et 1 ms.

Dans un mode de réalisation, les séquences comprennent une succession de 5 à 100 impulsions de courant, lesdites impulsions étant répétées à une fréquence comprise entre 1 Hz et 1 kHz.

Dans un mode de réalisation, la librairie de séquence contient des séquences personnalisées pour un patient.

L'invention concerne aussi une méthode de neurostimulation comprenant
- la sélection d'au moins deux séquences dans une librairie de séquences, chaque séquence comprenant une série temporelle d'impulsions de courant associée à une configuration de contacts électriques ; et
- l'application d'une activation neurale à au moins une électrode neurale comportant au moins deux contacts électriques et destinée à être implantée sur au moins un nerf, ladite activation neurale comprenant l'entrelacement des au moins deux séquences et l'adressage des impulsions de courant des au moins deux séquences vers les au moins deux contacts électriques par un répartiteur de courant.

Dans un mode de réalisation, la méthode de neurostimulation comprend en outre la répétition de l'application d'une activation neurale.

L'invention concerne enfin une activation neurale comprenant
- l'entrelacement d'au moins deux séquences choisies dans une librairie de séquences, chaque séquence comprenant une série temporelle d'impulsions de courant associée à une répartition de courant entre plusieurs contacts électriques ; et
- l'adressage des impulsions de courant des au moins deux séquences vers les au moins deux contacts électriques d'une électrode neurale destinée à être implantées sur un nerf.

### DÉFINITIONS

Dans la présente invention, les termes ci-dessous sont définis de la manière suivante :
**"Contact électrique"** concerne une surface d'une électrode neurale, cette surface étant au contact du nerf à stimuler et reliée électriquement au dispositif de stimulation neurale. Une électrode neurale peut comprendre plusieurs contacts électriques répartis sur différents anneaux.
**"Configuration de contacts électriques"** concerne une configuration dans laquelle chaque contact électrique d'une électrode neurale est associé à un rôle d'anode « a », de cathode « c » ou n'est pas connecté « - ».
**"Configuration de stimulation"** concerne une configuration dans laquelle sont définis, pour une configuration de contacts électriques donnée, les amplitudes des impulsions de courants qui sont envoyées dans chaque contact. La répartition des amplitudes des impulsions de courants est définie par un répartiteur de courant.
**"Electrode neurale"** concerne une électrode destinée à être implantée au contact d'un nerf afin d'y appliquer des stimuli électriques.
**"Entrelacement"** concerne la superposition temporelle de deux - ou plus - séries de signaux électriques. Ainsi, deux - ou plus - séquences d'impulsions de courant sont appliquées de manière alternée de telle sorte qu'une séquence débute avant qu'une autre séquence ne se termine. Les séquences d'impulsions de courant peuvent aussi être appliquées de manière composée, si les impulsions sont envoyées simultanément sur des configurations de contacts électriques différentes - *i.e.,* en l'absence de conflit de configuration qui verrait un même contact électrique mis en oeuvre avec des fonction différente (anode/cathode ou amplitudes différentes) pour les deux configurations - d'une même électrode neurale.
**"Impulsion de courant"** concerne un signal électrique bref, caractérisé par sa forme, son amplitude et sa durée. L'impulsion de courant est typiquement rectangulaire, mais peut adopter toute autre forme - triangulaire, asymétrique, biphasique... - selon les besoins de la stimulation neurale. L'amplitude de l'impulsion est typiquement comprise entre 10 µA et 10 mA. La durée de l'impulsion est typiquement comprise entre 5 µs et 1 ms. De plus, l'impulsion de courant peut être positive - injection de charges électriques - ou négative - retrait de charges électriques.
**"Stimulus biphasique"** concerne un stimulus comprenant deux phases, séparées par un temps d'interstimulation visant à maintenir la pré-polarisation induite par la première phase du stimulus biphasique. Le temps d'interstimulation est typiquement compris entre 25 µs et 150 µs.
**"Stimulus biphasique équilibré en charge"** concerne un stimulus biphasique pour lequel la quantité de charges mise en oeuvre lors de la première phase - charges injectées - est égale à la quantité de charges mise en oeuvre lors de la deuxième phase - charges retirées - les signes des charges étant toutefois opposés dans les deux phases. Au total, aucune charge nette n'est injectée ou retirée par un stimulus biphasique équilibré en charge. Les deux phases peuvent avoir des formes, amplitudes et durées différentes à condition que les quantités de charges soient identiques.

### DESCRIPTION DÉTAILLÉE

La présente invention concerne un dispositif de stimulation neurale.

Ce dispositif comprend au moins une électrode neurale destinée à être implantée sur au moins un nerf. Dans le cas particulier de stimulation neurale pour la restauration de la préhension, l'électrode neurale est implantée sur l'un des nerfs suivants : le nerf médian, le nerf ulnaire (ou cubital) ou le nerf radial. L'électrode neurale comporte au moins deux contacts électriques.

L'électrode neurale peut être choisie parmi les électrodes matricielles plates, destinées à être placée sur le nerf ; les électrodes intra fasciculaires, destinées à être placée à l'intérieur du nerf ; ou les électrodes gouttières (*cuff électrodes* en anglais) destinée à être placée autour du nerf. Les électrodes gouttières sont particulièrement adaptées car elles permettent de répartir régulièrement plusieurs contacts électriques tout autour du nerf. De manière avantageuse, les contacts électriques sont distribués sur l'électrode gouttière sur trois anneaux : deux anneaux extérieurs sur lesquels un seul contact électrique entoure complètement le nerf et un anneau intérieur sur lequel plusieurs contacts - typiquement de quatre à dix - sont répartis régulièrement. Les électrodes gouttières auto-ajustables permettent de répartir les contacts de l'anneau intérieur sur le nerf en s'adaptant à son diamètre.

Le dispositif de stimulation neurale comprend un générateur d'impulsions de courant. Ce générateur est connecté à l'électrode neurale par l'intermédiaire d'un répartiteur de courant. Le répartiteur de courant permet de répartir l'injection ou le retrait des charges électriques associées à l'impulsion de courant entre les différents contacts électriques de l'électrode neurale. Cette répartition est par la suite appelée une configuration de contacts électriques : elle contient à la fois les contacts électriques sélectionnés de l'électrode neurale et la répartition de courant entre ces contacts. De manière préférée, l'électrode neurale comprend une pluralité de configuration de contacts électriques. Ainsi, en répartissant les impulsions de courant sur différentes zones du nerf - à la surface du nerf pour une électrode gouttière - il est possible de stimuler de manière différente les fascicules du nerf, chaque fascicule contribuant au contrôle d'au moins un muscle.

De préférence, le répartiteur de courant peut amplifier l'impulsion électrique pour chaque contact électrique. Ainsi, le générateur d'impulsion de courant définit la forme, la durée et l'amplitude de référence de l'impulsion quand le répartiteur de courant définit l'amplitude réelle - après amplification - et la répartition des amplitudes réelles sur les différents contacts électriques. Cette répartition pondérée est par la suite appelée une configuration de stimulation.

Dans un mode de réalisation, les impulsions de courant sont des stimuli biphasiques équilibrés en charge. Dans ce cas, la forme de l'impulsion comprend deux phases : la première phase - dite de stimulation - est une injection de charge électriques et la deuxième phase - dite d'équilibrage - est un retrait de charges électriques. L'impulsion est équilibrée en charge lorsque la quantité de charge électrique injectée est égale à la quantité de charge électriques retirée, conduisant globalement à éviter l'accumulation de charges électriques sur le nerf stimulé et/ou sur les contacts électriques entre l'électrode neurale et le nerf stimulé. La première phase d'un stimulus biphasé équilibré en charge a une amplitude comprise entre 10 µA et 6 mA et une durée comprise entre 5 µs et 1 ms. La seconde phase est alors définie en amplitude et en durée du fait de l'équilibre en charge. Les deux phases peuvent être séparées par un temps d'interstimulation. De manière plus générale, les impulsions de courant peuvent être des stimuli multiphasiques - plusieurs phases d'injection de charges ou de retrait de charges - du moment que les quantités de charges injectées et de charges retirées demeurent égales.

Par exemple, le générateur de courant peut définir un stimulus biphasique équilibrée en charge comprenant une première phase rectangulaire d'amplitude de référence 100 µA et de durée 20 µs et une seconde phase rectangulaire d'amplitude de référence 50 µA et de durée 40 µs. Ensuite, le répartiteur de courant applique ce stimulus biphasique équilibrée entre les anodes : les deux anneaux extérieurs et les contacts 1 et 3 de l'anneau intérieur et la cathode : le contact 2 de l'anneau intérieur - les contacts 5 à 8 étant non connectés dans cette configuration de contacts électriques - d'une électrode gouttière dans une configuration de contact électriques TTR. De plus, l'impulsion est amplifiée d'un facteur 3 sur les anneaux extérieurs et sur les contacts 1 et 3 et amplifiée d'un facteur -12 sur le contact 2 - conduisant à un bilan d'amplification équilibré - et définissant une configuration de stimulation.

Le dispositif de stimulation neurale comprend en outre un moyen de stockage configuré pour contenir une librairie de séquences. Ce moyen de stockage peut être une mémoire incluse dans le dispositif de stimulation neurale ou un moyen de stockage externe en communication sans fil avec le dispositif de stimulation neurale. Une séquence est une série temporelle d'impulsions, dans laquelle une impulsion est répétée plusieurs fois. Cette répétition peut être strictement périodique : même impulsion répétée à une fréquence donnée, ou modulée. La modulation peut porter sur la fréquence - la période de répétition des impulsions est variable au cours de la séquence - l'amplitude - l'amplitude de chaque impulsion varie au cours de la séquence - et/ou la durée de l'impulsion. De plus, la séquence est associée à une configuration de contacts électriques définissant comment les impulsions de courant de la séquence doivent être répartis sur le nerf. Chaque séquence de cette librairie est associée à la stimulation d'un ou plusieurs fascicules d'un même nerf, dont l'effet est de contracter des muscles cibles conduisant à un mouvement complexe.

Enfin, le dispositif de stimulation neurale comprend un séquenceur configuré pour appliquer une activation neurale sur l'électrode neurale. Cette activation neurale comprend aux moins deux séquences entrelacées. Par entrelacement, on entend ici que la série temporelle des impulsions de courant appliquées sur l'électrode neurale est la superposition des séries temporelles d'impulsions de courant de chaque séquence. Toutefois, le dispositif de stimulation neurale ne peut mettre en oeuvre qu'une configuration de stimulation à la fois, il faut donc que l'entrelacement conduise à appliquer l'impulsion de courant d'une séquence entre deux impulsions de courant de l'autre séquence (ou des autres séquences). En particulier, l'application de deux séquences entrelacées correspond à définir une succession de configurations de stimulations sur l'électrode neurale, les configurations de stimulation étant associées à deux séquences différentes. Dans certains modes de réalisation, l'entrelacement peut concerner trois séquences, quatre séquences ou plus.

Pour entrelacer deux séquences, le séquenceur reçoit donc les informations de chacune des séquences - forme de signal, configuration de stimulation et intervalle/nombre de répétition du signal - et les applique en tenant compte des contraintes suivantes :
- les durées des intervalles des séquences sont identiques, ce qui permet d'intercaler le signal d'une séquence antre deux signaux d'une autre séquence,
- les configurations de stimulation sont compatibles, ou peuvent être modifiées entre les applications des signaux des différentes séquences.

Dans le cas où les muscles à solliciter pour un mouvement complexe agissent simultanément, les séquences à entrelacer comprennent le même nombre d'intervalles, ces intervalles ayant la même durée. En faisant débuter les séquences en même temps, les effets des stimulations neurales sont induits simultanément, conduisant par exemple à activer simultanément les muscles ciblés. Alternativement, si des muscles sont à solliciter dans un ordre précis, une première séquence peut débuter et une deuxième séquence être entrelacée plus tard dans la première. La première séquence peut se terminer avant la deuxième séquence. Ce principe s'applique aussi à trois, quatre ou plus de séquences.

Une activation neurale comprend donc plusieurs séquences dont les signaux sont appliqués en alternance. Une première séquence débute en appliquant une succession de signaux électriques séparées par des temps neutres - absence de signal -, puis une seconde séquence s'intercale pour appliquer des signaux électriques pendant les temps neutres de la première séquence. La seconde séquence débute donc avant que se termine la première séquence.

L'entrelacement des séquences peut aussi s'appuyer sur la modulation des temps neutres de chaque séquence, pour mieux gérer la contrainte d'intercalation des signaux. Par exemple, le séquenceur peut réduire les temps neutres des deux séquences de moitié, afin que le temps neutre total entre deux signaux successifs d'une même séquence soit conservé.

Par l'entrelacement de deux séquences, il est possible de stimuler par un seul et même nerf deux réponses simultanées et/ou en coopération. Dans le cas de réponses musculaires, cela conduit à un mouvement complexe synergique (coopération au mouvement) ou antagoniste (effet de stabilisation ou augmentation de la raideur articulaire) par exemple.

Dans un mode de réalisation, le dispositif de stimulation neurale comprend en outre un contrôleur configuré pour piloter une succession d'activations neurales. Ceci permet de créer une succession de mouvements complexes dont l'enchaînement conduit à la réalisation d'une fonction motrice, par exemple de l'ouverture de la main suivie d'une prise palmaire, restaurant ainsi la préhension. Alternativement, la succession d'activations neurales - à une fréquence déterminée - peut simplement conduire au maintien d'une - ou de plusieurs - contraction musculaire.

Dans un mode de réalisation, le contrôleur peut piloter le séquenceur afin de réaliser l'entrelacement des séquences. Dans ce cas, le contrôleur configure le séquenceur puis active les séquences et c'est au niveau du contrôleur que les contraintes d'applications des séquences sont vérifiées. Ce mode de réalisation est avantageux car il minimise les transferts d'information entre le contrôleur et le générateur de signaux électriques. En effet, le contrôleur configure le séquenceur, et le séquenceur s'appuie sur la librairie de séquences pour générer les signaux. Ainsi, on découple le contrôleur qui gère les stimulations neurales à haut niveau, en fonction des effets musculaires attendus, de la génération très précise des signaux - avec des précisions temporelles qui ne sont pas affectées par la transmission des données du contrôleur vers la génération des signaux.

Dans un mode de réalisation, les séquences comprennent une succession de 5 à 100 impulsions de courant, de préférence de 10 à 100 impulsions de courant. Ces impulsions de courant sont répétées à une fréquence comprise entre 1 Hz et 1 kHz, cette fréquence pouvant être éventuellement modulée. Dans le cas de stimulations efférentes (à visée motrice), les impulsions de courant sont répétées de préférence à une fréquence comprise entre 1 Hz et 250 Hz, typiquement entre 20 Hz et 100 Hz. La durée d'une séquence complète est très variable, en fonction de la tâche à réaliser. En particulier, pour maintenir une contraction musculaire, la séquence doit durer tout le temps du maintien de la contraction musculaire. De préférence, une séquence complète dure au moins 0.5 s et comprend au moins 10 impulsions.

Dans un mode de réalisation, le générateur d'impulsion est conçu pour contrôler très précisément les charges injectées ou retirées au niveau des électrodes neurales. Pour cela, le générateur d'impulsion peut s'appuyer sur une conception symétrique comprenant des sources de courant anodique et des sources de courant cathodiques configurées pour copier un courant de commande déterminé et pour que la somme des courants anodiques copiés soit égale à la somme des courants cathodiques copiés.

Dans un mode de réalisation, la librairie de séquence contient des séquences personnalisées pour un patient. En effet, les configurations de stimulation et les impulsions de courant qui permettent de stimuler efficacement un patient varient d'un patient à un autre. Il est donc nécessaire de définir les séquences personnalisées lors d'un processus d'apprentissage. Ce processus d'apprentissage peut se dérouler de la manière suivante.

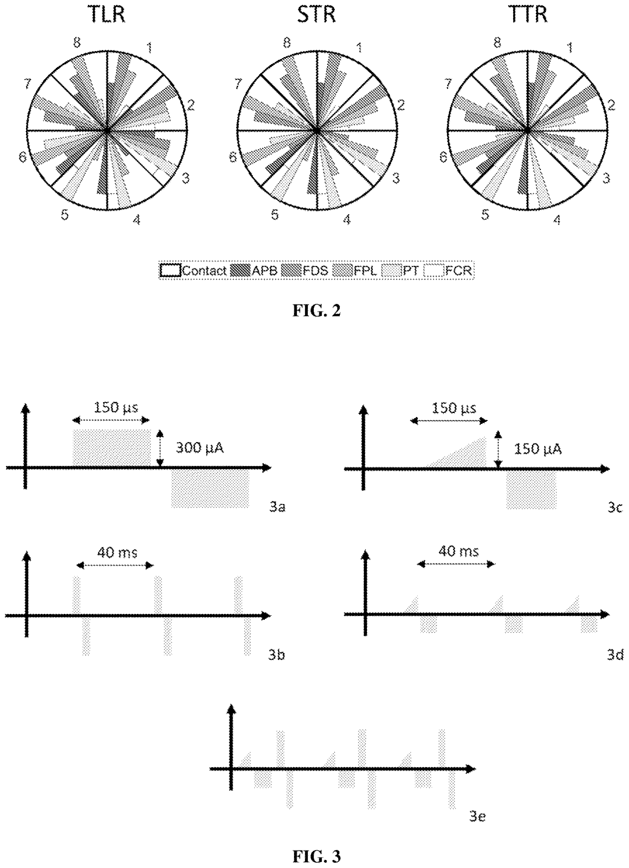

Après que le dispositif de stimulation neurale a été mis en place par l'implantation d'une électrode neurale sur un nerf d'un patient, des séquences modèles sont appliquées. Ces séquences modèles se définissent par les paramètres suivants : forme, amplitude et durée de l'impulsion de courant ; configuration de stimulation - contacts électriques employées, et amplification associée - ; nombre d'impulsions de courant et fréquence de répétition des impulsions de courant - définissant un intervalle entre deux signaux successifs. Une succession de séquence dans lesquelles les paramètres sont variés un à un permet d'identifier les seuils auxquels les muscles du patient agissent, permettant de construire une courbe de recrutement pour chacun d'eux. La figure 2 illustre pour un patient, avec une électrode gouttière implantée autour du nerf médian, pour des configurations de contacts électriques de type TLR, STR et TTR dont la cathode est définie successivement sur chacun des huit contacts électriques de l'anneau intérieur - chaque secteur correspond à l'un des contacts électriques pris comme cathode dans cette représentation graphique - , pour des séquences de stimulation de 15 répétitions à 25 Hz d'un stimulus biphasique équilibrée de durée 150 µs, les amplitudes réelles étant croissantes - après amplification par le séquenceur - de l'impulsion stimulant de manière sélective les muscles APB, FDS, FPL, PT ou FCR. On voit par exemple que la configuration de contacts électriques TLR permet de recruter avec l'intensité la plus basse (donc en premier) le muscle FPL pour les positions 6-7-8 de la cathode, les autres contacts permettant d'activer préférentiellement d'autres muscles. De plus chaque contact et chaque type de configuration (TLR, STR, TTR) permet d'obtenir une succession d'activation musculaire, et donc une synergie musculaire, différente. On peut ainsi par exemple obtenir une flexion des doigts puis du pouce pour obtenir une pince en stimulant uniquement le nerf médian avec une configuration recrutant préférentiellement les muscles FDS et FPL.

Ainsi, pour chaque muscle pris individuellement, et plus largement chaque enchaînement de contractions musculaires liées à l'augmentation de l'intensité, il est possible de définir la séquence qui permet d'activer une contraction musculaire synergique et fonctionnelle, pour un patient donné. Ensuite, sur la base des séquences personnalisées, il est possible d'entrelacer au moins deux séquences pour appliquer une stimulation neurale et provoquer un mouvement composé complexe, par exemple l'ouverture de la main et l'extension du poignet simultanée, puis de définir une succession d'activation neurales pour rétablir une fonction motrice, par exemple l'ouverture de la main puis la fermeture en mode pince.

Dans un mode de réalisation, l'ensemble du dispositif de stimulation est implantable et comprend en outre un moyen de communication sans fil. Ainsi, un patient peut être équipé lors d'une intervention chirurgicale du dispositif de stimulation. Ensuite, des commandes peuvent être transmises au séquenceur et/ou au contrôleur de dispositif de stimulation par un appareil de commande positionné au contact du patient - maintenu par un brassard par exemple - et contrôlé par le patient lui-même. Le patient peut activer l'appareil de commande de manière vocale, par l' activation volontaire de muscles équipés de détecteur électromyographiques, par la réalisation de mouvements volontaires stéréotypés détectés par des capteurs inertiels ou par une interface cerveau / machine, par exemple.

Dans un mode de réalisation, le dispositif de stimulation comprend au moins deux électrodes neurales, destinées à être implantées sur au moins deux nerfs différents. Ainsi, des stimulations complexes mettant en oeuvre des muscles activés par les différents nerfs équipés d'électrodes neurales peuvent être réalisées, par exemple pour obtenir la fermeture de la main sous la forme d'une pince (stimulation sur le nerf médian) et l'équilibre du poignet par son extension antagoniste (stimulation sur le nerf radial).

L'invention concerne également une méthode de neurostimulation, dans laquelle les caractéristiques décrites ci-dessus pour le dispositif de stimulation neurale s'appliquent.

Cette méthode comprend une étape de sélection dans une librairie de séquences de au moins deux séquences, chacune comprenant une série temporelle d'impulsions de courant associée à une configuration de contacts électriques. Dans une seconde étape, une activation neurale est appliquée à au moins une électrode neurale comportant au moins deux contacts électriques et destinée à être implantée sur au moins un nerf. Cette activation neurale résulte de l'entrelacement des au moins deux séquences et de l'adressage des impulsions de courant des au moins deux séquences vers les au moins deux contacts électriques par un répartiteur de courant.

Dans un mode de réalisation, la méthode de neurostimulation comprend en outre la répétition de l'application d'une activation neurale. Ainsi, plusieurs activations neurales, chacune résultant de l'entrelacement de au moins deux séquences, sont appliquées successivement.

L'invention concerne enfin une activation neurale, qui est une forme particulière de signal électrique. Les caractéristiques décrites ci-dessus pour le dispositif de stimulation neurale s'appliquent ici.

Cette activation neurale comprend d'une part l'entrelacement d'au moins deux séquences choisies dans une librairie de séquences, chaque séquence comprenant une série temporelle d'impulsions de courant associée à une répartition de courant entre plusieurs contacts électriques. Elle comprend d'autre part l'adressage des impulsions de courant des au moins deux séquences vers les au moins deux contacts électriques d'une électrode neurale destinée à être implantées sur un nerf.

En d'autres termes, une activation neurale est un signal électrique associé à une configuration de contacts électriques et qui permet d'obtenir une réponse, par exemple musculaire. Une activation neurale est une instruction élémentaire qui peut ensuite s'insérer dans une suite d'instructions permettant de réaliser des fonctions, en particulier des fonctions motrices.

Dans un mode de réalisation, l'adressage des impulsions de courant comprend les configurations de contacts électriques de la au moins une électrode neurale.

Dans un mode de réalisation, les impulsions de courant sont des stimuli biphasiques équilibrés en charge, de préférence avec la première phase ayant une amplitude comprise entre 10 µA et 6000 µA et une durée comprise entre 5 µs et 1 ms.

Dans un mode de réalisation, les séquences comprennent une succession de 1 à 100 impulsions de courant. Ces impulsions de courant sont répétées à une fréquence comprise entre 1 Hz et 1 kHz, cette fréquence pouvant être éventuellement modulée. Dans le cas de stimulations efférentes (à visée motrice), les impulsions de courant sont répétées de préférence à une fréquence comprise entre 1 Hz et 250 Hz, typiquement entre 20 Hz et 100 Hz. La durée d'une séquence complète est très variable, en fonction de la tâche à réaliser. En particulier, pour maintenir une contraction musculaire, la séquence doit durer tout le temps du maintien de la contraction musculaire. De préférence, une séquence complète dure au moins 0.5 s et comprend au moins 10 impulsions.

### DESCRIPTION DES FIGURES

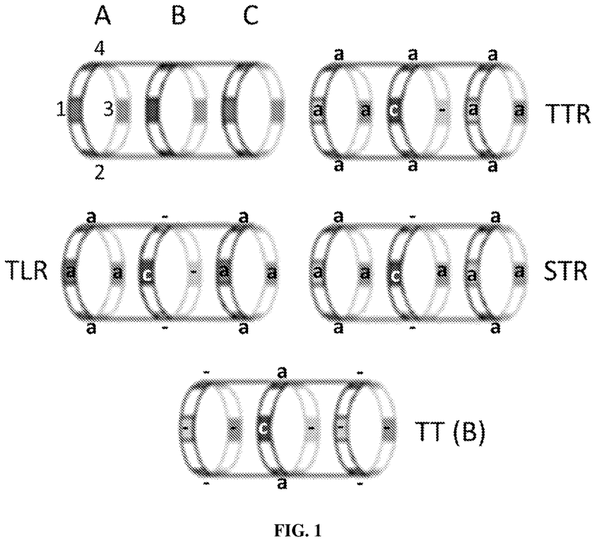

Figure 1 est une représentation d'une électrode neurale de type gouttière. Cette électrode (en haut à gauche) comprend trois anneaux A, B et C. Chaque anneau comporte quatre contacts électriques notées 1, 2, 3 et 4. Sur les anneaux extérieurs A et C, les quatre contacts sont reliés de sorte à former un seul potentiel électrique tout autour du nerf. Sur l'anneau intérieur B, les quatre contacts sont distincts et peuvent être connectés différemment. Les connexions anodiques sont notées « a », la connexion cathodique est notée « c » et les contacts électriques non connectés sont notés « - ». Dans la configuration de contacts électriques TLR, les anneaux extérieurs forment deux anodes et l'anneau intérieur comporte une cathode sur l'un de ses contacts électriques. Dans la configuration de contacts électriques TTR, la configuration de contacts électriques TLR est complétée par deux anodes situées sur l'anneau intérieur, immédiatement adjacentes à la cathode. Dans la configuration de contacts électriques STR, la configuration de contacts électriques TLR est complétée par une anode située sur l'anneau intérieur, diamétralement opposée à la cathode. Enfin, dans la configuration de contacts électriques TT, seul un anneau est utilisé (ici, l'anneau intérieur noté B, mais cela pourrait être un anneau extérieur A ou C) et comporte une cathode sur l'un de ses contacts électriques et deux anodes immédiatement adjacentes à la cathode.
Figure 2 représente l'ordre des recrutements musculaires pour un patient dans diverses configurations de contacts électriques ; cadran de gauche : TLR ; cadran central : STR ; cadran de droite : TTR. Chaque cadran contient huit secteurs correspondants aux huit contacts électriques de l'anneau intérieur de l'électrode neurale de type gouttière. Chaque secteur représente l'ordre de recrutement successif de chaque muscle (dès qu'il dépasse 10% de son recrutement) lorsque le contact électrique de l'anneau intérieur correspondant au secteur est utilisé comme cathode.
Figure 3 représente une activation neurale résultant de l'entrelacement de deux séquences, chaque séquence comprenant des impulsions de courant sous forme de stimuli biphasiques équilibrées.

### MODES DE RÉALISATION ILLUSTRATIFS DE L'INVENTION

La présente invention se comprendra mieux à la lecture des exemples suivants qui illustrent non-limitativement l'invention.

Un patient souffrant de tétraplégie a été opéré afin de lui implanter deux électrodes gouttières, l'une sur le nerf médian et l'autre sur le nerf radial. Les électrodes gouttière avaient la structure décrite en Figure 1, avec deux anneaux extérieurs et un anneau central disposant de 8 contacts électriques pour le nerf médian et de 6 contacts électriques pour le nerf radial. Les configurations de contact électrique employées sont les configurations TTR (tripolaire transverse), TLR (tripolaire longitudinale) et STR (steering current - pilotage de courant), représentées sur la figure 1.

Afin de créer une librairie de séquences personnalisée pour ce patient ; différentes configurations de stimulations - configuration de contacts, amplitudes des impulsions et séquence - ont été appliquées et les réponses musculaires mesurées via des dispositifs de mesure électromyographiques, par analyse d'images des mouvements provoqués ou par mesure des forces exercées par les muscles stimulés. Ces mesures ont permis de définir des courbes de recrutement pour chaque muscle. Ainsi, ont été déterminées les réponses induites par le nerf médian de :
- *flexor carpi radialis* (**FCR**) responsable de la flexion du poignet,
- *pronator teres* (**PT**) responsable de la pronation de l'avant-bras et du poignet,
- *flexor digitorum superficialis* (**FDS**) responsable de la flexion des doigts (sauf le pouce)
- *flexor pollicis longus* (**FPL**) responsable de la flexion du pouce, et
- *abductor pollicis brevis* (**APB**) responsable de l'abduction du pouce.

Par ailleurs, ont été déterminées les réponses induites par le nerf radial de
- *extensor carpi radialis* (**ECR**) responsable de l'extension du poignet,
- *extensor pollicis longus* (**EPL**) responsable de l'extension du pouce, et
- *extensor digitorum communis* (**EDC**) responsable de l'extension des doigts.

La figure 2 représente la succession des recrutements musculaires lorsque l'intensité de stimulation augmente pour une configuration de contacts électriques - formant une configuration de stimulation - permettant de recruter chaque muscle : recrutement de APB, FDS, FPL, PT et FCR dans les configurations de contacts TLR, STR et TTR. Ces graphiques associés aux courbes de recrutement de chaque muscle permettent d'identifier, pour une configuration de contact donnée, les niveaux à partir desquels les dits muscles sont recrutés et l'ordre dans lequel ces dits muscles sont activés. Il est alors possible de définir des séquences personnalisées pour le patient, ces séquences permettant d'activer sélectivement un muscle ou plusieurs muscles simultanément.

La figure 3 représente de manière simplifiée deux séquences de la librairie. Un stimulus biphasique équilibrée en charge et symétrique - figure 3-a, première phase d'amplitude 300 µA et de durée 150 µs et seconde phase identique, mais de signe opposé, après un temps d'interstimulation de 25 µs - est répétée à une fréquence de 25 Hz - période de 40 ms - pour former une première séquence - figure 3-b - de 15 impulsions. Un stimulus biphasique équilibrée en charge - figure 3-c, première phase d'amplitude linéairement croissante jusqu'à 150 µA et de durée 150 µs et seconde phase d'amplitude 150 µA et de durée 75 µs mais de signe opposé, après un temps d'interstimulation de 25 µs - est répétée à une fréquence de 25 Hz - période de 40 ms - pour former une deuxième séquence - figure 3-d - de 15 impulsions. Chaque séquence permet de recruter successivement de un à plusieurs muscles du patient, lorsqu'elle est associée à une configuration de contacts et une amplification particulière, définie par la courbe de recrutement correspondante. Il s'agit donc d'induire des synergies d'actions musculaires différentes. Ces synergies peuvent ensuite être combinées grâce à l'entrelacement des séquences sur un ou plusieurs nerfs.

Afin de coordonner plusieurs mouvements musculaires et d'obtenir une fonction motrice, les deux séquences sont entrelacées, comme illustré figure 3-e. Il faut noter que les impulsions successives sont appliquées par des configurations de contact différentes, mais qu'après chaque impulsion, aucune charge nette n'a été injectée au patient, du fait de la nature équilibrée en charge des impulsions.

L'application de la stimulation neurale de ces deux séquences entrelacées conduit à des mouvements complexes qui ne peuvent pas être obtenus pas application successive de deux séquences. En effet, si les séquences sont appliquées successivement, les muscles activés par une séquence vont se relaxer lorsque cette séquence s'achève et ne pourront pas agir en synergie avec les muscles activés par la séquence suivante.

## Revendications

1. Dispositif de stimulation neurale comprenant
• au moins une électrode neurale comportant au moins deux contacts électriques et destinée à être implantée sur au moins un nerf ;
• un générateur d'impulsions de courant connecté à la au moins une électrode neurale par l'intermédiaire d'un répartiteur de courant ;
• un moyen de stockage configuré pour contenir une librairie de séquences, chaque séquence comprenant une série temporelle d'impulsions de courant associée à une configuration de contacts électriques ; et
• un séquenceur configuré pour appliquer une activation neurale à la au moins une électrode neurale, ladite activation neurale comprenant au moins deux séquences entrelacées de la librairie de séquences.

2. Dispositif de stimulation neurale selon la revendication **1,** dans lequel les séquences sont entrelacées de manière alternée de telle sorte qu'une desdites séquences entrelacées débute avant qu'une autre desdites séquences entrelacées ne se termine.

3. Dispositif de stimulation neurale selon la revendication **1** ou **2,** dans lequel les séquences à entrelacer comprennent le même nombre d'intervalles, ces intervalles ayant la même durée.

4. Dispositif de stimulation neurale selon l'une quelconque des revendications **1** à **3,** comprenant en outre un contrôleur configuré pour piloter une succession d'activations neurales.

5. Dispositif de stimulation neurale selon la revendication **4,** dans lequel le contrôleur configure le séquenceur puis active les séquences à entrelacer.

6. Dispositif de stimulation neurale selon l'une quelconque des revendications **1** à **5,** dans lequel la au moins une électrode neurale comprend une pluralité de configurations de contacts électriques.

7. Dispositif de stimulation neurale selon l'une quelconque des revendications **1** à **6,** dans lequel les impulsions de courant sont des stimuli biphasiques équilibrés en charge et préférentiellement dont la première phase a une amplitude comprise entre 10 µA et 6000 µA et une durée comprise entre 5 µs et 1 ms.

8. Dispositif de stimulation neurale selon l'une quelconque des revendications **1** à **7,** dans lequel les séquences comprennent une succession de 5 à 100 impulsions de courant, lesdites impulsions étant répétées à une fréquence comprise entre 1 Hz et 1 kHz.

9. Dispositif de stimulation neurale selon l'une quelconque des revendications **1** à **8,** dans lequel la librairie de séquence contient des séquences personnalisées pour un patient.

## Patentansprüche

1. Nervenstimulationsvorrichtung, umfassend
- mindestens eine Nerven-Elektrode, die mindestens zwei elektrische Kontakte umfasst und dazu bestimmt ist, auf mindestens einem Nerv implantiert zu werden;
- einen Stromimpulsgenerator, der mittels eines Stromverteilers mit der mindestens einen Nerven-Elektrode verbunden ist;
- ein Speichermittel, das so konfiguriert ist, dass es eine Sequenzbibliothek enthält, wobei jede Sequenz eine Zeitreihe von Stromimpulsen umfasst, die einer Konfiguration von elektrischen Kontakten zugeordnet ist; und
- einen Sequenzer, der so konfiguriert ist, dass er eine Nervenaktivierung an die mindestens eine Nerven-Elektrode anlegt, wobei die Nervenaktivierung mindestens zwei verschachtelte Sequenzen aus der Sequenzbibliothek umfasst.

2. Nervenstimulationsvorrichtung nach Anspruch **1,** wobei die Sequenzen alternierend derart verschachtelt sind, dass eine der verschachtelten Sequenzen beginnt, bevor eine andere der verschachtelten Sequenzen endet.

3. Nervenstimulationsvorrichtung nach Anspruch **1** oder **2,** wobei die zu verschachtelnden Sequenzen die gleiche Anzahl von Intervallen umfassen, wobei diese Intervalle die gleiche Dauer aufweisen.

4. Nervenstimulationsvorrichtung nach einem der Ansprüche **1** bis **3,** die weiter eine Steuerung umfasst, die so konfiguriert ist, dass sie eine Abfolge von Nervenaktivierungen steuert.

5. Nervenstimulationsvorrichtung nach Anspruch **4,** wobei die Steuerung den Sequenzer konfiguriert und anschließend die zu verschachtelnden Sequenzen aktiviert.

6. Nervenstimulationsvorrichtung nach einem der Ansprüche **1** bis **5,** wobei die mindestens eine Nerven-Elektrode eine Vielzahl von Konfigurationen von elektrischen Kontakten umfasst.

7. Nervenstimulationsvorrichtung nach einem der Ansprüche **1** bis **6,** wobei die Stromimpulse ladungsausgeglichene zweiphasige Reize sind und bevorzugt deren erste Phase eine Amplitude im Bereich zwischen 10 µA und 6000 µA und eine Dauer im Bereich zwischen 5 µs und 1 ms aufweist.

8. Nervenstimulationsvorrichtung nach einem der Ansprüche **1** bis **7,** wobei die Sequenzen eine Abfolge von 5 bis 100 Stromimpulsen umfassen, wobei die Impulse mit einer Frequenz im Bereich zwischen 1 Hz und 1 kHz wiederholt werden.

9. Nervenstimulationsvorrichtung nach einem der Ansprüche **1** bis **8,** wobei die Sequenzbibliothek für einen Patienten personalisierte Sequenzen enthält.

## Claims

1. A neural stimulation device comprising:
• at least one neural electrode comprising at least two electrical contacts and intended to be implanted on at least one nerve;
• a current pulse generator connected to the at least one neural electrode via a current distributor;
• a storage element configured to contain a library of sequences, each sequence comprising a time series of current pulses associated with a configuration of electrical contacts; and
• a sequencer configured to apply neural activation to the at least one neural electrode, said neural activation comprising at least two interleaved sequences of the library of sequences.

2. The neural stimulation device according to claim **1,** wherein the sequences are interleaved alternately such that one of said sequences begins before another of said sequences ends.

3. The neural stimulation device according to claim **1** or **2,** wherein the sequences to be interleaved comprise the same number of intervals, these intervals having the same duration.

4. The neural stimulation device according to any one of claims **1** to **3,** further comprising a controller configured to drive a succession of neural activations.

5. The neural stimulation device according to claim **4,** wherein the controller configures the sequencer and then activates the sequences to be interleaved.

6. The neural stimulation device according to any one of claims **1** to **5,** wherein the at least one neural electrode comprises a plurality of electrical contact configurations.

7. The neural stimulation device according to any one of claims **1** to **6,** wherein the current pulses are load-balanced biphasic stimuli and wherein the first phase has preferably an amplitude of between 10 µA and 6000 µA and a duration of between 5 µs and 1 ms.

8. The neural stimulation device according to any one of claims **1** to **7,** in which the sequences comprise a succession of 5 to 100 current pulses, the said pulses being repeated at a frequency comprised between 1 Hz and 1 kHz.

9. The neural stimulation device according to any one of claims **1** to **8,** wherein the sequence library contains sequences personalized for a patient.
